# EUROPEAN PATENT APPLICATION

(11) **EP 1 908 771 A1**
(43) Date of publication of application: **09.04.2008**
(21) Application number: 06123046.2
(22) Date of filing: 26.10.2006
(51) Int. Cl.: C07K 14/195, G01N 33/68

(54) **Thermophilic sulfate-binding protein, its use and a biosensor comprising the protein**

(30) Priority: 04.10.2006 EP 06121737
(71) Applicant: Humboldt-Universität zu Berlin, D-10099 Berlin (DE)
(72) Inventor: Schneider, Erwin, 12205 Berlin (DE); Scheffel, Frank, 10439 Berlin (DE)
(74) Representative: Ziebig, Marlene

(57) **Abstract**

The invention relates to a substantially pure sulfate-binding protein derived from *Alicyclobacillus acidocaldarius.* Another object of the invention concerns the use of the protein for determining the presence or the concentration of sulfate in a sample as well as methods for that. The invention also relates to a biosensor comprising that sulfate-binding protein.

## Description

The invention relates to a substantially pure sulfate-binding protein derived from *Alicyclobacillus acidocaldarius.* Another object of the invention concerns the use of the protein for determining the presence or the concentration of sulfate in a sample as well as methods for that. The invention also relates to a biosensor comprising that sulfate-binding protein.

Sulfate is an important chemical substance in both environmental and biological systems. It is one of several major environmental contaminants, which is especially to be found in waters. In biological systems, hydrogen sulfide is one of the by-products of sulfate assimilation. The toxicity of excess hydrogen sulfide on living organisms is known. In humans, it can cause olfactory paralysis and sudden loss of consciousness.

There are various chemical and physical methods available for the determination of sulfate, such as titrimetric, gravimetric, colorimetric, spectrophotometric, Raman and IR methods as well as ion chromatography and ion selective electrode-based methods. However, these methods are cumbersome, time-consuming and lack selectivity and sensitivity in the micromolar range.

It is also shown that biosensors are capable of detecting a particular analyte. In general, a molecule of biological origin serves as the biorecognition element that selectively binds the analyte, thereby producing an analytical signal that is proportional to the analyte concentration. For example, suitable molecules of biological origin can be found among the periplasmic binding proteins of *E. coli.* Sensing systems for maltose, glucose, phosphate and metal ions are known in the prior art, using their respective binding proteins. WO 03/060464 A2 discloses such a reagentless sensing system for measuring carbohydrates based on the galactose/glucose-binding protein of *E. coli.*

Shrestha et al. (2002) Biotechnology and Bioengineering 78(5), 517-526, teach the design of fluorescently labeled sulfate-binding protein (SBP) of *E. coli.* Several mutants were evaluated for probe attachment and eventually employed in the development of a fluorescent-based biosensing system for sulfate. However, the application of the biosensor is restricted to a neutral pH value and room temperature. The sensitivity does not fall below the micromolar range and selenate even interferes in sensing sulfate. The sensor also exhibits a low stability requiring storage at 4°C.

There is still a need for valuable information on pollution levels as well as on the purity of drinking water. Furthermore, the accurate determination and quantitation of sulfate in biological systems is important. None of it can be sufficiently satisfied by the prior art technology.

Therefore, the technical problem forming the basis of the present invention is to provide a further binding substance, which guarantees a sensitive and reliable binding of sulfate, especially under stringent conditions.

The present invention solves this problem by providing a substantially pure sulfate-binding protein derived from *Alicyclobacillus acidocaldarius* or variants, mutants or parts of said protein having at least 80 % homologous sequences and the same function.

Surprisingly, it has been found that the bacterium *Alicyclobacillus acidocaldarius* contains a sulfate-binding protein, which is termed CysP_{AA} herein. The underlying gene *cysP_{AA}* is localized within an operon structure of an ABC sulfate importer. The CysP_{AA} protein is capable of binding sulfate with high affinity and expressed at the cell surface. It was therefore unexpected to find a sulfate transporter of the ABC type in the organism since CysP_{AA} as an essential component is fully exposed to the generally unfavorable acidic environment. CysP_{AA} exhibits a certain tertiary structure that is found to bind the respective ligand sulfate in a specific and selective manner. In the presence of sulfate, the binding protein undergoes a conformational change in its global structure to accommodate sulfate inside the binding site. In addition to its unique sensitivity, the inventors have demonstrated the remarkable resistance of the protein to harsh environmental conditions. Neither pH shifts into the extreme acidic milieu nor high temperatures exceeding 70°C affect the stability of CysP_{AA} unfavorably. As used herein, the term stability denotes the overall characteristics of the protein, which are associated with its proper functional appearance, and summarizes features such as solubility, globular structure, sulfate-binding capability, etc. For example, the soluble status comes along with the correct protein folding, which make sulfate binding not only possible but represent essential prerequisites for persistent high affinity.

CysP_{AA} can be expressed *in-vivo* in a natural or recombinant form. The bacterium *Alicyclobacillus acidocaldarius* is deposited under the account ATCC27009 and can be ordered accordingly. *A. acidocaldarius* grows best about pH 3.5 and at temperatures about 60°C considering thermo acidic conditions. Downstream processing follows known standard procedures. For example, affinity chromatography can be performed using a sulfate-presenting matrix.

CysP_{AA} is preferably overexpressed due to a enhanced synthesis rate and less purification efforts. The *cysP_{AA}* gene is inserted into an expression vector that is either commercially available, accessible to the public or can be constructed from available plasmids by routine use of published methods. The choice of vector in accordance to the expression system, the cloning of the *cysP_{AA}* gene into said vector containing at least one restriction site and the control of expression by a suited promoter and/or operator and terminator is performed by standard techniques known to those skilled in the art. An expression strain, such as *E. coli* or derivatives thereof, is transformed and the bacterial cells are cultivated under appropriate conditions. The process parameters, comprising composition of the nutrient solution, process period, temperature, pH, gassing, stirrer speed, etc. are favorably pre-determined in test experiments and scaled up to the fermenter. The protein can be easily purified by means of a tag that is encoded by the vector or introduced therein during cloning in order to express a fusion construct. Several tags have been developed to purify overexpressed recombinant proteins. Among others, they share the features of a minimal effect on tertiary structure and biological activity as well as the applicability to a number of different proteins. Preferably, CysP_{AA} is overexpressed *in E. coli* as N-terminal fusion with a His₆ peptide tag and purified by metal ion affinity chromatography resulting in 6-10 mg CysP_{AA} per liter culture medium.

In addition to the cell-based expression, the cell-free system is applicable for protein biosynthesis. Cell-free systems involve coupled transcription and translation reactions *in-vitro,* which are based on a cell lysate (generally called S30). The lysate is pre-produced by means of cell disruption and subsequent centrifugation at 30.000 g. The advantages of the cell-free protein biosynthesis include, *inter alia,* the solely dependence of protein expression on the template, high yields as well as the possibility to incorporate unusual amino acids into the protein.

Recombinant expression techniques are favorably applied in order to generate variants, mutants or parts of CysP_{AA} having at least 80 % homologous sequences and the same function, wherein the parts have at least 40 % of the CysP_{AA} size. However, it is to be understood that any derivative may also result from a spontaneous alteration. Derivatives preferably exhibit the same performance as the wild-type or a better one. In detail, the sulfate-binding activity corresponds to that of the wild-type or is even higher trained. Alternatively or additionally, the derivatives may be adapted to run under special environmental conditions.

A couple of methods are known to the skilled artisan to generate equivalent CysP_{AA} proteins, i.e. proteins that are analog in function to those of the inventive teaching. Therefore, the invention also contains the mentioned derivatives. For example, mutants can be generated by substitution, deletion, insertion, translocation, inversion and/or addition of at least a single amino acid. The deletion can be also excluded to distinguish the mutant form the part in the meaning of the invention. It is known that certain amino acids exhibit similar physicochemical characteristics making the substitution among each other possible. Variants of CysP_{AA} can arise from modifications, such as alkylation, arylation or acetylation of at least a single amino acid, from incorporation of enantiomers and/or from fusion of the CysP_{AA} with a single or multiple amino acids, a peptide or a protein. It is preferred in the meaning of the invention that CysP_{AA} is fused to a purification tag for affinity chromatography. Parts of CysP_{AA} relates to a restriction to those regions that are sufficient for the expression of a specific function. All alterations are inevitably limited by the requirement of preserving the function. However, the parts of the CysP_{AA} can be small due to the characterization of the binding pocket that is sufficient for sulfate binding. In the meaning of the invention, it is to be distinguished between functional CysP_{AA} parts of any size (having 100 % identity as to their length) and homologous sequences which homology is related to the entire protein. Preferably, the homology between wild-type CysP_{AA} and a variant or mutant thereof having the same features amounts to at least 80 %, more preferably 90 %, most preferably 95 %. Similarly, the homology is to be considered if the aforementioned part of any size is altered to a variant or mutant. The size of the CysP_{AA} parts is preferably at least 50 % of the wild-type CysP_{AA} size, more preferably at least 89 %, most preferably at least 95 %. In addition, several techniques are described in the prior art to generate non-homologous peptides with the same function. All peptide derivatives, which are developed on the basis of the present ingredients by such procedures, are covered by the present teaching if solving the problem of the invention.

The binding capability of CysP_{AA} is described by the affinity to sulfate which is expressed in terms of the dissociation constant. In an embodiment of the present invention, the wild-type protein has a dissociation constant of less than 500 µM, preferably less than 100 µM, more preferably 0.1 µM to 50 µM, most preferably 1.7 to 2.9 µM. The filtration method was performed to determine the dissociation constant. It shall be understood that other methods and/or conditions as well as variants, mutants or parts of CysP_{AA} protein or at least 80 % homologous sequences having the same function may exhibit differing constants, particularly lower values up to the nanomolar range.

The protein of the invention has sulfate-binding activity at pH 9.0 or less, preferably at pH 1.6 to 7.0, more preferably at pH 3.5 to 6.0. Furthermore, the protein is stable at a temperature of 100°C or less, preferably 4°C to 90°C. More preferably, the maximum temperature lies between 60°C and 80°C, which is tolerated without denaturation by CysP_{AA}. The combination of both parameters reveals outstanding protein stability at a pH range of 1.6 to 7.0 and at temperatures below 80°C, preferably at a pH range of 3.5 to 6.0 and at a maximum temperature of 60°C to 80°C. The stability is reflected by the solubility of the protein and maintenance of sulfate-binding activity. Homologous SBP from *Salmonella typhimurium* or *E. coli* showing approximately 95 % identity among each other are completely denatured and precipitated after heating to 70°C at pH 5.0 to 6.0 for 30 minutes. Contrary to that, CysP_{AA} remains stable in solution. In addition to in the thermo acidic resistance, the sensitivity is clearly enhanced in comparison to conventional methods for sulfate measurement.

In a preferred embodiment of the present invention, said protein comprises the amino acid sequence of SEQ ID NO: 1 or variants, mutants or parts of the sequence or at least 80 % homologous sequences having the same function. CysP_{AA} is composed of 330 amino acids in length and has a molecular weight of 35.6 kDa. The identity to the homologous *E. coli* SBP amounts to approximately 36 %. The theoretical isoelectric point is calculated to be 5.9.

CysP_{AA} consists of two globular domains and the sulfate-binding cleft between them. One molecule of sulfate has 8 potential electrostatic interactions (2 per oxygen atom) involving Ser 134, Gly175, Gly135, Ser50, Tyr193, Gly136, and Ser15. The resultant conformational change that accompanies sulfate binding forms the basis of the protein application in a sensing system. From the structure residues suitable for covalent modification can be predicted. Such residues are not part of the binding pocket but undergo a conformational change upon sulfate binding. By attaching a reporter that is sensitive to the local environment to the correct site of CysP_{AA}, it is possible to perform measurements of sulfate by monitoring the signal or change in the emitted signal of the reporter moiety. When using a fluorophore for labeling CysP_{AA}, an embodiment that is preferred according to the present invention, it is important that the fluorophore is covalently attached to a site and at a position where maximum conformational change occurs in order to obtain maximal signal versus background fluorescence. Fluorescent techniques are well-known in the art. Nevertheless, it is also possible to employ other detection methods instead or in addition to fluorescence.

Preferably, labeling cysteine residues with sulfhydro-specific probes enables the quantitation of the conformational change, which can be related to the amount of sulfate present in a solution. Wild-type SBP lacks cysteine residues in its structure. Hence, in another preferred embodiment of the invention, the amino acid sequence of SEQ ID NO: 1 is modified to contain at least a single cysteine residue. Such cysteine residues can be introduced by site-directed mutagenesis employing PCR. The presence of a unique cysteine provides site-specific labeling of the protein, thereby reducing the background signal observed with the random labeling. Furthermore, the incorporation of a unique cysteine on the protein molecule permits selection of a specific site and allows for the optimization of the induced signal or signal change of the labeled protein in the presence of sulfate. By studying the X-ray crystal structure of CysP_{AA} in the sulfate-bound form and free form, appropriate sites can be rationally selected for site-specific mutagenesis to cysteine.

According to the invention, it is particularly preferred to substitute a lysine moiety by a cysteine moiety at position 193. The complete sequence of this mutant is listed in SEQ ID NO: 2.

Any cysteine mutant, preferably that of SEQ ID NO: 2, can be labeled with the thiol-reactive fluorescent probe N-[2-(1-maleimidyl)ethyl]-7-(diethylamino)coumarin-3-carboxamide (MDCC). MDCC consists of a flexible spacer arm between its maleimide group and the coumarin, which allows the probe to rotate freely in response to the environmental change that occurs as a consequence of a change in protein conformation. Coumarin derivative fluorescent probes are known to respond efficiently to changes in the local environment. In addition to MDCC, other thiol-reactive fluorescent probes, such as 6-acryloyl-2-dimethylaminonaphthalene (acrylodan), 5-((((2-iodoacetyl)amino)ethyl)amino)-naphthalene-1-sulfonic acid (1,5-IAEDANS) and N-((2-iodoacetoxy)ethyl)-N-methyl)amino-7-nitrobenz-2-oxa-1,3-diazole (IANBD ester) can also be explored as potential probes to label cysteine mutants. Acrylodan, a naphthalene derivative probe, is similar in structure to MDCC but lacks a spacer arm. 1,5-IAEDANS is also a naphthalene derivative fluorescent probe but with a reactive iodoacetamide group and contains an ethylene spacer arm. These fluorescent probes are specific for attachment to cysteine residues and sensitive to the polarity of their environment.

It is preferred that the fluorophore is coupled at cysteine via a maleimide group. Maleimide moieties are introduced to facilitate the attachment of the label to free thiols of cysteine residues to the protein.

The present invention also relates to a nucleic acid sequence, which encodes the sulfate-binding protein according to the invention. Furthermore, the invention concerns a vector including the nucleic acid sequence according to the invention, particularly to a bacterial vector or a virus.

Object of the present invention is also the use of the sulfate-binding protein according to the invention, the nucleic acid according to the invention or the vector according to the invention for detecting sulfate in a sample and/or determining the concentration of sulfate in a sample. The sulfate-binding capability of CysP_{AA} and derivatives thereof is utilized to catch the ligand in a given sample if present. The binding event is visualized by any signal, which is generated as response of the system upon complex formation. Preferably, the binding is monitored via fluorescence. Steady-state fluorescence studies indicate that the hinge motion and binding properties of CysP_{AA} can be used with fluorophores for biosensing sulfate reagentless. Subsequently, calibration plots are construed by relating the changes in the signal with the amount of ligand present. It shall be understood that the isolated or vector-borne nucleic acid sequence will be translated into the sulfate-binding protein before using it.

Another object of the invention is a method for detecting sulfate in a sample comprising the steps of contacting the sample with the protein according to the invention and detecting a binding event between the protein and sulfate. Preferably, sulfate is detected up to the submicromolar range, more preferably to the nanomolar range.

The sample is provided from an environmental system comprising soil, water or air, preferably drinking water or wastewater. The sample to be analyzed can also be taken form a biological system, i.e. an organisms of any origin. Body fluids are easily taken from a mammal, preferably in terms of blood, serum, plasma, saliva and urine. Furthermore, products of biological origin can be analyzed, such as food, beverages and the like. The detection method of the invention can also be applied for quality control of plain chemical system, such as medicaments, purity grade of chemicals, etc.

The contacting step is performed in solution. The sample is favorably provided as aqueous solution, whereas the protein is immobilized onto a solid phase.

In a preferred embodiment of the invention, the protein is labeled with a reporter moiety that generates a signal or a change in signal upon a conformational change in the protein when bound with sulfate. The change in signal is a change in the signal intensity and/or the signal lifetime. It does not matter whether the change in signal results in a decrease or increase of the signal. Even the loss of any signal is regarded as change in signal.

Although any assay method may be used for monitoring the signal, the binding event is preferably detected by luminescence, UV/VIS coloring, electrochemical processes or radioactive emission. More preferably, photoluminescence is applied.

Luminescence concerns the emission of light as a result of chemiluminescence, bioluminescence or photoluminescence. Chemiluminescence involves the emission of visible light as a result of a chemical reaction, whereas bioluminescence requires the activity of luciferase. The presently preferred photoluminescence, which is also known as fluorescence stimulation, is caused by the absorption of photons, preferably provided by radiation, which is released again as photon with a shift in wavelength of 30 to 50 nm and within a period of approximately 10⁻⁸ seconds. The instruments for fluorescence detection include, but are not limited to typical benchtop fluorometers, fluorescence multi-well plate readers, fiber optic fluorometers, fluorescence microscopes and microchips/microfluidics systems coupled with fluorescence detection.

VIS coloring denotes the visualization of any achromatic substance in order to be visible to the naked eye. Preferably, the intensity of coloring is measured by a photometer.

Electrochemical probes are also well-established and described in WO 03/060464 A2, for example, which is incorporated by reference herein.

Radioactive radiation of isotopes is measured by scintillation. The process of liquid scintillation involves the detection of beta decay within a sample via capture of beta emissions in a system of organic solvents and solutes referred to as the scintillation cocktail. The beta decay electron emitted by radioactive isotopes such as ³H, ¹⁴C, ³²P, ³³P and ³⁵S in the sample excites the solvent molecule, which in turn transfers the energy to the solute. The energy emission of the solute (the light photon) is converted into an electrical signal by a photo-multiplier tube within a scintillation counter. The cocktail must also act as a solubilizing agent keeping a uniform suspension of the sample. Gamma ray photons often arise as a result of other decay processes (series decay) to rid the newly formed nucleus of excess energy. They have no mass and produce little if any direct ionization by collision along their path. Gamma photons are absorbed for detection and quantitation by one or more of three mechanisms: The Compton effect, the photoelectric effect and pair production. A favorable gamma decay isotope of the present invention is ¹²⁵I.

It is another object of the present invention to determine the concentration of sulfate in a sample by a method comprising the steps of contacting the sample with the protein according to the invention, detecting a binding event between the protein and sulfate, and correlating the amount of signal or change in signal with the amount of sulfate in the sample. Therefore, the protein of the invention is incubated with various concentrations of sulfate first. Upon sulfate binding to CysP_{AA}, a change in the conformation of the protein is induced. The amount of emitted signal or change in signal observed in the presence of sulfate is indicative of the local environmental change experienced by the probe. The change can be then related to the concentration of sulfate in the sample. Using this method, the inventors demonstrated sensitivity to submicromolar or even nanomolar concentrations. The calibration plot reveals that the method can be applied in a dynamic range that spans over two orders of magnitude, preferably three orders of magnitude, more preferably four orders of magnitude.

It is to be understood that the prior teaching of the present specification concerning the CysP_{AA} protein and its use is considered as valid and applicable without restrictions to the methods for determining the presence or concentration of sulfate in a sample if it is expedient.

Still another object of the invention relates to a biosensor comprising the protein of the invention, wherein said protein is immobilized onto a solid support, further a reporter moiety or a reporter apparatus, and a means for detecting a signal of the reporter moiety or the reporter apparatus generated upon binding of sulfate to the protein. Such a biosensor combines high selectivity and rapid release of the analyte to ensure reversibility of the sensor response.

The immobilization of the protein onto a solid support affords the possibility of using the CysP_{AA}-based biosensor in a number of applications. The nature of the surface can be diverse, e.g. silica, a hydrogel, a sol-gel, a polymer film, a membrane, self-assembling monomers, a Langmuir-Blodgett film, methacrylate, polypropylene, PDMS, chitin, resin, etc. Depending on the choice of surface, the chemistry for anchoring of the protein to the surface will be different. Various methods for the conjugation and immobilization of protein to insoluble substrates are well-known in the art, wherein the usage of a spacer molecule is preferred. For example, a cysteine can be introduced at the protein's N- or C-terminus using commercially available molecular biology reagents. The cysteine residue may be subsequently anchored onto a gold-containing surface, or one can take advantage of the reactivity of the sulfhydro group on the cysteine to create a covalent bond between the SH- and a group on the surface, such as a maleimide group or an iodoacetamide group. The N- or C-terminal cysteine is to be distinguished from another cysteine residue for attachment of a signal-generating molecule.

A suitable apparatus in the meaning of the invention transforms the energy of interaction between receptor (CysP_{AA}) and ligand (sulfate) into an electrical signal. For example, electrochemical, optical, calorimetric or piezo-electric transducers are applied, preferably field effect transistors (FET). FETs are electronic components controlling the current in the semiconductor material by the electrical field at the gate. The binding of molecules to the gate can vary the electrical field. Changes in dipole moments or a charge accumulation at immobilized biomolecules, which are caused by ligands, are monitored by Bio-FET.

Preferably, the protein is labeled with the reporter moiety as already described in the course of the present specification. It is further preferred that the biosensor comprises a means for correlating the signal of the reporter moiety with the amount of sulfate present in the sample. The prior teaching of the present specification concerning the CysP_{AA} protein, its use and the methods for determining the presence or concentration of sulfate in a sample is considered as valid and applicable without restrictions to the biosensor if it is expedient.

Further, the invention may be practiced as a kit comprising the protein of the invention, the nucleic acid sequence of the invention or the vector of the invention, particularly in order to perform the inventive method. Thus, the invention includes an article that comprises written instructions or directs the user to written instructions for how to practice the method of the invention or use the sensors of the invention. In an embodiment, the kit further comprises a reporter moiety or a reporter apparatus, preferably a fluorophore or a field-effect transistor. The prior teaching of the present specification concerning the CysP_{AA} protein, its use, e.g. in biosensors, and the methods for determining the presence or concentration of sulfate in a sample is considered as valid and applicable without restrictions to the kit if it is expedient.

The present invention also relates to an amino acid sequence comprising the SEQ ID NOs: 1 or 2.

In the scope of the present invention, a substantially pure sulfate-binding protein derived from *Alicyclobacillus acidocaldarius* or variants, mutants or parts thereof are provided. The CysP_{AA} sequence is disclosed for the first time herein. The inventive protein is characterized by a high stability under high-temperature conditions and very acidic pH surroundings. Therefore, it outmatches homologous SBP from mesophiles in respect of their resistance while maintaining the sulfate-binding activity or outperforming it into nanomolar ranges. These characteristics form the basis for a reproducible recognition of sulfate. The CysP_{AA} protein is cost-efficiently overexpressed, purified and even easily modified. The binding efficiency of the cysteine mutant according to the invention is not affected by the mutagenesis. Such a modification opens up an access for labeling or immobilization, which is essentially required for the application of the protein in sulfate sensing systems. In particular, a biosensor is constructed for determining the presence or concentration of sulfate in a sample. According to the favorable features of CysP_{AA}, the biosensor demonstrates reliability and consistency, a long life span and low manufacturing costs. It can be applied either in the daily lab routine but also in the analytical practice of extreme outdoor habitats or samples thereof, respectively.

It is to be understood that this invention is not limited to the particular methods, uses or biosensors described herein, as such matter may, of course, vary. It is also to be understood that the terminology used herein is for the purpose of describing particular embodiments only and is not intended to limit the scope of the present invention, which is only defined by the appended claims. As used herein, including the appended claims, singular forms of words such as "a," "an," and "the" include their corresponding plural referents unless the context clearly dictates otherwise. Thus, e.g., reference to "a sequence" includes one or more different sequences and reference to "a method" includes reference to equivalent steps and methods known to a person of ordinary skill in the art, and so forth. Unless otherwise defined, all technical and scientific terms used herein have the same meaning as commonly understood by a person of ordinary skill in the art to which this invention belongs.

Although methods and materials similar or equivalent to those described herein can be used in the practice or testing of the present invention, suitable examples are described below. The following examples are provided by way of illustration and not by way of limitation. Within the examples, standard reagents and buffers that are free from contaminating activities (whenever practical) are used.

### BRIEF DESCRIPTION OF THE DRAWINGS

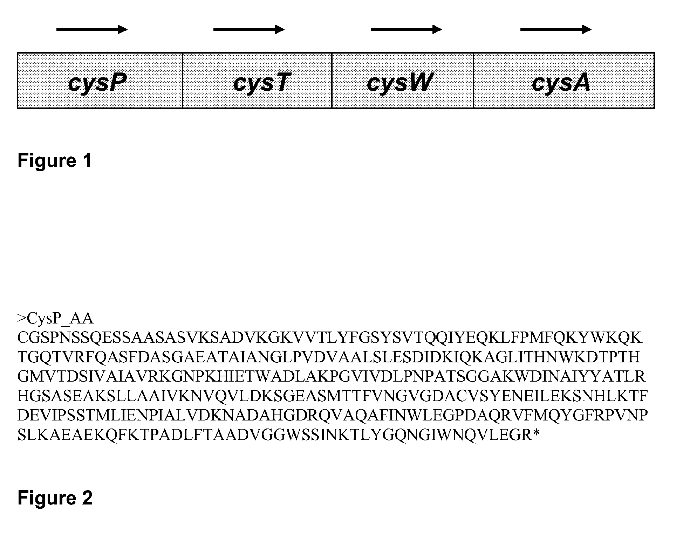

Fig. 1 shows the *cysP_{AA}* localization on the bacterial chromosome. The gene *cysP_{AA}* is arranged within an operon structure comprising further genes for an ABC sulfate importer.
Fig. 2 shows the amino acid sequence of CysP_{AA} according to SEQ ID NO: 1.

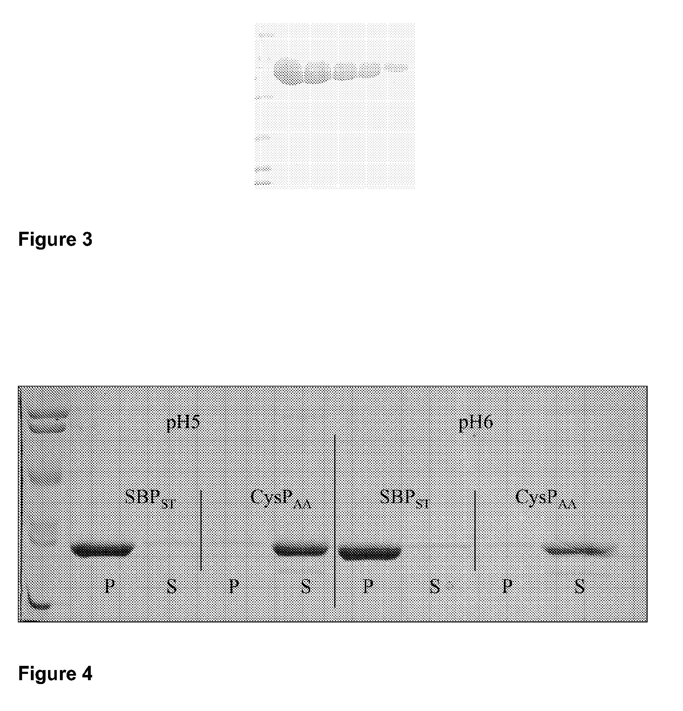

Fig. 3 shows the course of CysP_{AA} protein purification. The SDS gel is loaded with several elution fractions of a Ni-NTA affinity chromatography.
Fig. 4 shows stability studies of *A. acidocaldarius* CysP_{AA} in comparison to *S. typhimurium* SBP. The SDS gel is loaded with protein samples from precipitate (P) and supernatant (S) obtained after heating the proteins to 70°C at pH 5.0 and 6.0, respectively, for 30 minutes.

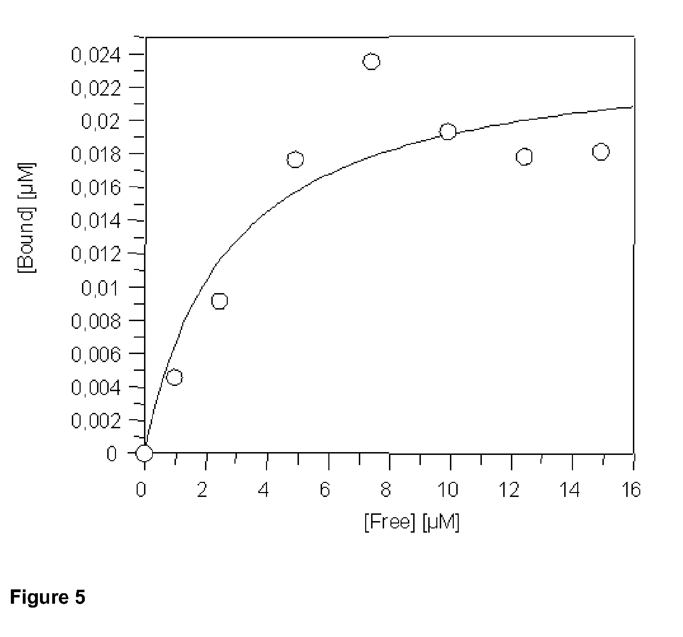

Fig. 5 shows the sulfate-binding curve of CysP_{AA} determined at pH 3.6 and 60°C.

### EXAMPLES

### Isolation and sequencing of cysP_{AA}

The *cysP* gene was cloned from genomic DNA by chromosome walking. For expression the gene lacking its signalpeptide-encoding-fragment was amplified by PCR with the primers cysP-7 (forward) and cysP-8 (reverse) (5' -ATCGAAG**GATCC**TCAGCGCCCCTCCAACACTTGA TTCC'-3 and 5'-TTGGTC**GGATCC**GGCCGGATCGCCCAATTCCTCGCAGGAG-3') and inserted as a BamHI fragment into expression vector pET15b (Novagene), yielding pFR01. DNA sequencing was carried out commercially by MWG (Ebersberg).

### Cloning and purification of CysP_{AA}

For overexpression the *cysP* gene was amplified by the method of PCR, using plasmid pFR01 as template. The primers were designed to amplify the sequence encoding a N-terminal truncated version of the mature protein. The oligonucleotides cysP-9 (forward) 5'-ATCGCC**CATATGAA**GGTTGTCACGC-TCTATTTCGGGTCC-3' with a Nde1 site (in bold) and cysP-7 (reverse) 5'-ATCGAA**GGATCC**TCAGCGCCCCTCCAACACTTGATTCC-3' with a BamHI site were used as 5'and 3'primers respectively. The amplified fragment encoded CysP starting with K25 of the mature protein. It was cloned directly as a Ndel-BamHI fragment into the corresponding sites of pET15 (Novagen), yielding pFSA66. This resulted in a translated protein with a 6xhis-tag and a thrombin site fused to its N-terminus.
Cells were grown in Luria-Bertani broth at 28°C and overexpression was induced with 0,5 mM IPTG at A₆₅₀ = 0,5 and growth was continued for 3 hours.
CysP was purified from the cytosolic fraction of *E. coli* strain Rosettall<plysS><pFSA66> by affinity chromatography.
All purification steps were carried out at 4°C. Cell disruption was carried out with a french pressure cell in a solution containing 50 mM MOPS-KOH (pH 7), 150 mM NaCl, 5% glycerol, 0,1 mM PMSF (buffer A) and DNase I. Cell debris was removed by ultracentrifugation and the resulting supernatant was incubated with talon resin (Clonetech) for 45 minutes. The resin was transferred to a column and washed with buffer A containing 20 mM imidazole. CysP was eluted with 250 mM imidazole in the same buffer. The fractions containing the recombinant protein were pooled and incubated with thrombin-agarose (Sigma) overnight at room-temperature for removal of the his-tag. The buffer of the cleaved protein was exchanged on a PD10 column (G.E. Health Care) to buffer D (20 mM acetate buffer, pH 5,0, 20 mM NaCl, 1 % glycerol). The protein was then concentrated using a cetrifugal filter device (Centricon YM-10, Millipore). The final protein concentration was determined with the BCA protein assay reagent (Pierce) with bovine serum albumine as a standard.

### Stability assay

Temperature and pH stability was determined by incubating CysP_{AA} and SBP (each at 0,13 mg/ml), respectively, at the indicated temperature and pH in citrate (pH 1,6-2,6) or citrate-phosphate (pH 3,6-8) buffer for 30 min. After centrifugation (15 min, 13000rpm) protein in the resuspended pellet and the supernatant was visualized on an SDS-gel.

### Sulfate binding assay and determination of the dissociation constant

Binding of [³⁵S]sulphate to purified CysP was performed by the Method of Richarme & Kepes (1983). Standard assay mixtures (100 µl) containing 100 mM acetate buffer, pH 3,6 and 5 µM CysP were preheated at 60°C for 1 min prior to the addition of [³⁵S]sulphate (G.E. Health Care, 0,32 mCi/mg). After 1 min, the reaction was terminated by adding 2 ml of an ice-cold saturated (NH₄)₂SO₄ solution and immediately filtered through a nitrocellulose filter (0,45 µM). The filter was washed once with the same solution, followed by distilled water, and retrained radioactivity was determined in a liquid scintillation counter. To determine the binding constant, CysP was subjected to a denaturation/renaturation procedure using guanidine hydrochloride (6M) to remove excess bound unlabeled substrate (Hall *et al.,* 1998).

### X-ray structure analysis of CysP_{AA}

CysP_{AA} was concentrated to 15 mg/ml and crystals were grown at 18°C using the hanging drop vapor diffusion method. The reservoir solution contained 0.1 M Tris, pH 8.5, 0.2 M Na-acetate and 34% PEG 3350. Drops were prepared by mixing 1 ml of reservoir and 1 ml of protein solution (20 mM Na-acetate pH 5.0, 20 mM NaCl, 1 % glycerol) at 14 mg/ml. Platelet-like crystals formed within 48 hours and reached their final size in 3 - 5 days. Optically flawless crystals were briefly soaked in a cryoprotection medium of 0.1 M Tris, pH 8.5, 0.2 M Na-acetate, 34% PEG 3350 and 10 % v/v glycerol, mounted in a nylon loop and then flash-cooled in liquid N₂. X-ray diffraction data were recorded at Bessy II.

### In vitro mutagenesis of cysP_{AA}

Mutations were introduced in the *cysP* gene with the QuickChange mutagenesis Kit by the manufactures protocol.

### Labeling of the cysteine mutant and fluorescence studies

CysP_{AA}K193C/C210S was conjugated in 50 mM Bis-Tris, pH 7,0 with a four-fold molar excess of MIANS for 15 min on ice. The reaction was stopped with 1mM DTT. Unbound label was removed by washing the mixture in a centricon centrifugal filter device (YM 10). For the fluorescence studies using fluorometer SFM25 (Kontron Instruments) a sample volume of 1 ml was used in a quartz cuvette. The excitation wavelength was set 340 nm and emission was detected from 350-500 nm. A fixed concentration of protein (17,5 µg) was incubated with sulphate for 5 min at 60°C.

## Claims

1. A substantially pure sulfate-binding protein derived from *Alicyclobacillus acidocaldarius* or variants, mutants or parts of said protein having at least 80 % homologous sequences and the same function.

2. Protein according to claim 1 wherein said protein comprises the amino acid sequence of SEQ ID NOs: 1 or 2 or variants, mutants or parts of the sequences or at least 80 % homologous sequences having the same function.

3. Protein according to claim 1 or 2 wherein said protein is labeled with a reporter moiety, preferably a fluororphore.

4. Use of the protein according to one of claims 1 to 3 for detecting sulfate in a sample and/or determining the concentration of sulfate in a sample.

5. A method for detecting sulfate in a sample comprising the steps of:
- contacting the sample with the protein according to one of claims 1 to 3, and
- detecting a binding event between the protein and sulfate.

6. The method according to claim 5 wherein the protein is labeled with a reporter moiety, preferably a fluorophore moiety, which generates a signal or a change in signal upon a conformational change in the protein when bound with sulfate.

7. A method for determining the concentration of sulfate in a sample comprising the steps according to claim 5 followed by the further step of:
- correlating the amount of signal or change in signal with the amount of sulfate in the sample.

8. A biosensor comprising
- the protein according to one of claims 1 to 3, wherein said protein is immobilized onto a solid support,
- a reporter moiety or a reporter apparatus, and
- a means for detecting a signal of the reporter moiety or the reporter apparatus generated upon binding of sulfate to the protein.

9. The biosensor according to claim 8 further comprising a means for correlating the signal of the reporter moiety with the amount of sulfate present in a sample.

10. Amino acid sequence comprising the SEQ ID NOs: 1 or 2.


SEQUENCE LISTING

<110> Humboldt-Universität zu Berlin

<120> Thermophilic sulfate-binding protein, its use and a
      biosensor comprising the protein

<130> P436006EP1

<140>
<141>

<160> 6

<170> PatentIn Ver. 2.1

<210> 1
<211> 330
<212> PRT
<213> Alicyclobacillus acidocaldarius

<400> 1
Cys Gly Ser Pro Asn Ser Ser Gln Glu Ser Ser Ala Ala Ser Ala Ser
  1               5                  10                  15

Val Lys Ser Ala Asp Val Lys Gly Lys Val Val Thr Leu Tyr Phe Gly
             20                  25                  30

Ser Tyr Ser Val Thr Gln Gln Ile Tyr Glu Gln Lys Leu Phe Pro Met
         35                  40                  45

Phe Gln Lys Tyr Trp Lys Gln Lys Thr Gly Gln Thr Val Arg Phe Gln
     50                  55                  60

Ala Ser Phe Asp Ala Ser Gly Ala Glu Ala Thr Ala Ile Ala Asn Gly
 65                  70                  75                  80

Leu Pro Val Asp Val Ala Ala Leu Ser Leu Glu Ser Asp Ile Asp Lys
                 85                  90                  95

Ile Gln Lys Ala Gly Leu Ile Thr His Asn Trp Lys Asp Thr Pro Thr
            100                 105                 110

His Gly Met Val Thr Asp Ser Ile Val Ala Ile Ala Val Arg Lys Gly
        115                 120                 125

Asn Pro Lys His Ile Glu Thr Trp Ala Asp Leu Ala Lys Pro Gly Val
    130                 135                 140

Ile Val Asp Leu Pro Asn Pro Ala Thr Ser Gly Gly Ala Lys Trp Asp
145                 150                 155                 160

Ile Asn Ala Ile Tyr Tyr Ala Thr Leu Arg His Gly Ser Ala Ser Glu
                165                 170                 175

Ala Lys Ser Leu Leu Ala Ala Ile Val Lys Asn Val Gln Val Leu Asp
            180                 185                 190

Lys Ser Gly Glu Ala Ser Met Thr Thr Phe Val Asn Gly Val Gly Asp
        195                 200                 205

Ala Cys Val Ser Tyr Glu Asn Glu Ile Leu Glu Lys Ser Asn His Leu
    210                 215                 220

Lys Thr Phe Asp Glu Val Ile Pro Ser Ser Thr Met Leu Ile Glu Asn
225                 230                 235                 240

Pro Ile Ala Leu Val Asp Lys Asn Ala Asp Ala His Gly Asp Arg Gln
                245                 250                 255

Val Ala Gln Ala Phe Ile Asn Trp Leu Glu Gly Pro Asp Ala Gln Arg
            260                 265                 270

Val Phe Met Gln Tyr Gly Phe Arg Pro Val Asn Pro Ser Leu Lys Ala
        275                 280                 285

Glu Ala Glu Lys Gln Phe Lys Thr Pro Ala Asp Leu Phe Thr Ala Ala
    290                 295                 300

Asp Val Gly Gly Trp Ser Ser Ile Asn Lys Thr Leu Tyr Gly Gln Asn
305                 310                 315                 320

Gly Ile Trp Asn Gln Val Leu Glu Gly Arg
                325                 330


<210> 2
<211> 330
<212> PRT
<213> Artificial Sequence

<220>
<223> Description of Artificial Sequence:
      sulfate-binding protein

<400> 2
Cys Gly Ser Pro Asn Ser Ser Gln Glu Ser Ser Ala Ala Ser Ala Ser
  1               5                  10                  15

Val Lys Ser Ala Asp Val Lys Gly Lys Val Val Thr Leu Tyr Phe Gly
             20                  25                  30

Ser Tyr Ser Val Thr Gln Gln Ile Tyr Glu Gln Lys Leu Phe Pro Met
         35                  40                  45

Phe Gln Lys Tyr Trp Lys Gln Lys Thr Gly Gln Thr Val Arg Phe Gln
     50                  55                  60

Ala Ser Phe Asp Ala Ser Gly Ala Glu Ala Thr Ala Ile Ala Asn Gly
 65                  70                  75                  80

Leu Pro Val Asp Val Ala Ala Leu Ser Leu Glu Ser Asp Ile Asp Lys
                 85                  90                  95

Ile Gln Lys Ala Gly Leu Ile Thr His Asn Trp Lys Asp Thr Pro Thr
            100                 105                 110

His Gly Met Val Thr Asp Ser Ile Val Ala Ile Ala Val Arg Lys Gly
        115                 120                 125

Asn Pro Lys His Ile Glu Thr Trp Ala Asp Leu Ala Lys Pro Gly Val
    130                 135                 140

Ile Val Asp Leu Pro Asn Pro Ala Thr Ser Gly Gly Ala Lys Trp Asp
145                 150                 155                 160

Ile Asn Ala Ile Tyr Tyr Ala Thr Leu Arg His Gly Ser Ala Ser Glu
                165                 170                 175

Ala Lys Ser Leu Leu Ala Ala Ile Val Lys Asn Val Gln Val Leu Asp
            180                 185                 190

Cys Ser Gly Glu Ala Ser Met Thr Thr Phe Val Asn Gly Val Gly Asp
        195                 200                 205

Ala Cys Val Ser Tyr Glu Asn Glu Ile Leu Glu Lys Ser Asn His Leu
    210                 215                 220

Lys Thr Phe Asp Glu Val Ile Pro Ser Ser Thr Met Leu Ile Glu Asn
225                 230                 235                 240

Pro Ile Ala Leu Val Asp Lys Asn Ala Asp Ala His Gly Asp Arg Gln
                245                 250                 255

Val Ala Gln Ala Phe Ile Asn Trp Leu Glu Gly Pro Asp Ala Gln Arg
            260                 265                 270

Val Phe Met Gln Tyr Gly Phe Arg Pro Val Asn Pro Ser Leu Lys Ala
        275                 280                 285

Glu Ala Glu Lys Gln Phe Lys Thr Pro Ala Asp Leu Phe Thr Ala Ala
    290                 295                 300

Asp Val Gly Gly Trp Ser Ser Ile Asn Lys Thr Leu Tyr Gly Gln Asn
305                 310                 315                 320

Gly Ile Trp Asn Gln Val Leu Glu Gly Arg
                325                 330


<210> 3
<211> 38
<212> DNA
<213> Artificial Sequence

<220>
<223> Description of Artificial Sequence: primer cysP-7

<400> 3
atcgaaggat cctcagcgcc cctccaacac ttgattcc                          38


<210> 4
<211> 40
<212> DNA
<213> Artificial Sequence

<220>
<223> Description of Artificial Sequence: primer cysP-8

<400> 4
ttggtcggat ccggccggat cgcccaattc ctcgcaggag                        40


<210> 5
<211> 39
<212> DNA
<213> Artificial Sequence

<220>
<223> Description of Artificial Sequence:  primer cysP-9

<400> 5
atcgcccata tgaaggttgt cacgctctat ttcgggtcc                              39


<210> 6
<211> 38
<212> DNA
<213> Artificial Sequence

<220>
<223> Description of Artificial Sequence: primer cysP-7

<400> 6
atcgaaggat cctcagcgcc cctccaacac ttgattcc                               38